(19) Europäisches Patentamt
European Patent Office
Office européen des brevets

(11) Veröffentlichungsnummer: **0 007 008**
**B1**

(12) # EUROPÄISCHE PATENTSCHRIFT

(45) Veröffentlichungstag der Patentschrift: **06.01.82**

(51) Int. Cl.³: **C 07 C 69/653,** C 07 C 67/10

(21) Anmeldenummer: **79101872.4**

(22) Anmeldetag: **11.06.79**

(54) **Verfahren zur Herstellung von Halogenbutenylacrylaten.**

(30) Priorität: **22.06.78 DE 2827323**

(43) Veröffentlichungstag der Anmeldung:
**23.01.80 Patentblatt 80/2**

(45) Bekanntmachung des Hinweises auf die Patenterteilung:
**06.01.82 Patentblatt 82/1**

(84) Benannte Vertragsstaaten:
**BE DE FR GB IT NL**

(56) Entgegenhaltungen:
**SU-A-226 593**
**SU-A-226 596**
**SU-A-571 476**
**US-A-3 255 163**

(73) Patentinhaber: **BAYER AG, Zentralbereich Patente, Marken und Lizenzen, D-5090 Leverkusen 1, Bayerwerk (DE)**

(72) Erfinder: **Beck, Manfred, Dr., Auf dem Krahwinkel 5, D-5068 Odenthal (DE)**

**EP 0 007 008 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung von Halogenbutenylacrylaten

Die Erfindung betrifft ein Einstufenverfahren zur Herstellung von Halogenbutenylacrylaten durch Umsetzung von gegebenenfalls substituierten Acrylsäuresalzen mit 1,4-Dihalogenbuten-(2) in einem Zweiphasensystem.

Aus der US-PS 3 255 163 ist ein Verfahren zur Herstellung von Halogenbutenylacrylaten bereits bekannt. Die Verbindungen erhält man, indem Acrylsäureester in Gegenwart von 4-Halogen-2-butenolen einer Umesterungsreaktion unterworfen werden. Dieses Verfahren arbeitet jedoch mit einem hohen Überschuß an Acrylsäureester, dessen Aufarbeitung schwierig und verlustreich ist. Weiterhin ist das Ausgangsprodukt 4-Halogen-2-butenol-(1) nur in einer verlustreichen Mehrstufensynthese herzustellen. Das beschriebene Verfahren ist daher technisch unvorteilhaft.

Aus Synthesis 1974, Seite 867 ist es weiterhin bekannt, Dibromalkenyle mit Kaliumacetat zu entsprechenden Bisacetaten umzusetzen. Die Herstellung entsprechender Monoverbindungen wird jedoch nicht beschrieben. Ebensowenig gibt es Hinweise zur Reaktion von ungesättigten Carbonsäuren mit Dihalogenalkenylen unter Bildung von Monosubstitutionsprodukten. Zur Durchführung des beschriebenen Verfahrens werden hohe Mengen eines Phasentransfer-Katalysators, nämlich 22 Mol-%, bezogen auf das Halogenalkyl benötigt, so daß hier kaum noch von einer Katalyse gesprochen werden kann.

Beide Halogenatome des 1,4-Dihalogenbuten-2 besitzen eine hohe Reaktionsfähigkeit. Als Beispiel sei die schnelle Verseifbarkeit angeführt. Setzt man 1,4-Dichlorbuten-2 mit Natriumcarbonat bei 100° C um, isoliert man nur ein Gemisch zweier isomerer Butendiole (Pudovik, Ž. Obšč. Chim. 19 (1949) 1185, CA. 1950, 1005).

Aufgrund der hierdurch nachgewiesenen leichten Verseifbarkeit beider Halogenatome im 1,4-Dichlorbuten-2 mußte daher bei der Herstellung von entsprechenden Chlorbutenylestern in alkalisch-neutralem wäßrigem Milieu mit einer Hydrolyse zum 4-Hydroxybutenylester gerechnet werden. Diese Reaktion tritt überraschenderweise nicht ein.

Es wurde nun gefunden, daß Acrylsäureester des 4-Halogen-2-butenols-(2) in einem Einstufenverfahren durch Reaktion von gegebenenfalls substituierten Acrylsäuren mit 1,4-Dihalogenbuten-(2) erhalten werden können. Bei diesem Verfahren arbeitet man in einem Zweiphasensystem. In der wäßrigen Phase befindet sich das Alkali- oder Erdalkalisalz der Acrylsäure, die organische Phase besteht aus dem im Überschuß eingesetzten Dihalogenbuten, gegebenenfalls verdünnt mit einem Lösungsmittel.

Die Reaktion kann durch Verwendung eines Phasentransfer-Katalysators aus der Gruppe der quartären Ammonium-Phosphonium oder Sulfoniumsalze beschleunigt werden. Sie verläuft nach folgendem Schema:

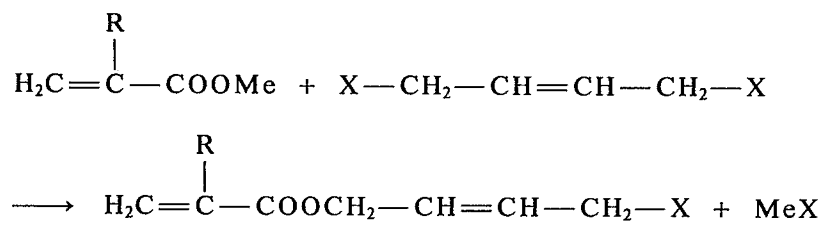

R = H, $C_1-C_4$-Alkyl
X = Halogen wie z. B. Cl, Br, I
Me = Ammonium, Alkali, Erdalkali (unter Berücksichtigung der Wertigkeit des Metall-Ions)

Die Erfindung betrifft somit ein Verfahren zur Herstellung von Halogenbutenylacrylaten der Formel (I)

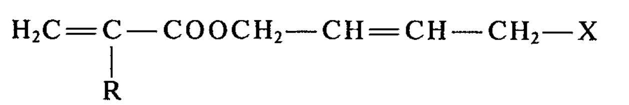

(I)

in der

R Wasserstoff oder einen Alkylrest mit 1 bis 4 Kohlenstoffatomen und
X ein Halogenatom darstellt, das dadurch gekennzeichnet ist, daß man pro Mol eines Salzes der Acrylsäure der Formel (II)

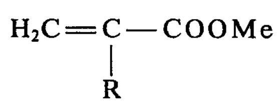

(II)

in der

R Wasserstoff oder einen Alkylrest mit 1 bis 4 Kohlenstoffatomen und
Me ein Ammonium, ein Alkali- oder Erdalkalimetallion darstellt,

1 bis 7 Mole an 1,4-Dihalogenbuten-(2) in einem Zweiphasensystem bei Temperaturen von 10 bis 140° C miteinander umsetzt.

Als Alkylsubstituenten R seien beispielsweise Methyl, Äthyl, Propyl, Isopropyl, Butyl, Isobutyl und tert.-Butyl, vorzugsweise Methyl, erwähnt.

Als Halogensubstituenten X kommen Chlor, Brom, Jod, bevorzugt Chlor, in Betracht.

Als gegebenenfalls verwendbares Lösungsmittel für das Dihalogenbuten können mit Wasser nicht mischbare Lösungsmittel, bevorzugt aliphatische oder aromatische Kohlenwasserstoffe wie Hexan, Cyclohexan, Toluol oder Xylol eingesetzt werden.

Als Alkali- und/oder Erdalkalimetall seinen Natrium, Kalium, Magnesium, Calcium, Strontium, Barium, besonders bevorzugt Natrium oder Kalium, erwähnt.

Die Acrylsäuren werden mit Dihalogenbuten umgesetzt, indem man bevorzugt mit einem Überschuß von 2 bis 5 Mol an 1,4-Dihalogenbuten pro Mol Acrylsäure arbeitet. Diese Verfahrensweise hat den Vorteil, daß man wahlweise auch ohne Lösungsmittel arbeiten kann. Bei dieser bevorzugten Verfahrensweise erleichtert sich die Aufarbeitung. Die beiden Phasen werden nach der Reaktion voneinander getrennt und die organische Phase wird einer Fraktionierung unterworfen. Sie gestaltet sich besonders einfach, da das überschüssige Dichlorbuten wegen seines relativ niedrigen Siedepunktes in einer ersten Kolonne leicht abgetrennt werden kann. In einer zweiten Kolonne wird der Ester als Seitenstrom in hoher Reinheit erhalten. Man erhält die Ester in Ausbeuten von 60—78% bezogen auf die eingesetzte Säure und von 70—80%, bezogen auf Dichlorbuten. Das überschüssige Dichlorbuten wird vollständig zurückgewonnen.

Das neue Verfahren hat gegenüber dem bekannten Verfahren ferner den Vorteil, daß billige verdünnte Acrylsäure verwendet werden kann, wie sie bei technischen Verfahren anfällt. Außerdem ist kein Überschuß dieser Verbindung erforderlich. Eine Wiederverwendung der nicht-umgesetzten Acrylsäure in der wäßrigen Phase ist möglich.

Die Reaktion erfolgt durch Erhitzen einer gerührten zweiphasigen Mischung aus dem Salz der Acrylsäure gelöst in Wasser und der Halogenverbindung. Der Mischung können außerdem Phasentransfer-Katalysator ggf. verschiedene bekannte Polymerisationsinhibitoren zugesetzt werden, beispielsweise Hydrochinon N-Nitrosodiphenylamin, p-tert.-Butylbrenzcatechin, 4-Methoxyphenol, Phenothiazin oder Gemische von zwei oder mehreren Stabilisatoren. Die Umsetzung erfolgt bevorzugt bei Temperaturen im Bereich von 60 bis 100° C, besonders bevorzugt im Bereich von 80 bis 95° C.

Als gegebenenfalls anwesende Phasentransfer-Katalysatoren sind geeignet quartäre Ammonium-, Phosphonium- und Sulfoniumverbindungen. Ihre allgemeine Formel lautet $RR'R''R'''E^+X^-$, in der die Reste R, R', R'', R''' aliphatische oder aromatische Reste, die gegebenenfalls hydroxylsubstituiert sein können, sind und E ein Stickstoff- oder Phosphoratom bedeuten. X ist ein Anion. Die allgemeine Formel der Sulfoniumverbindungen lautet $RR'R''S^+X^-$ mit der gleichen Bedeutung der Reste.

Bevorzugt werden Ammoniumverbindungen eingesetzt. Beispiele für letztere sind:

Tetrabutylammoniumhalogenid
Tetraoctylammoniumhalogenid
Octyl-tributylammoniumhalogenid
Octadecyltributylammoniumhalogenid
Octyl-bis-(hydroxyäthyl)-benzylammoniumhalogenid
Dodecyl-bis-(hydroxypropyl)-benzylammoniumhalogenid
Octadecyl-dimethyl-benzylammoniumhalogenid

Der Katalysator wird in einer Menge von 0,01 bis 10 Mol-% eingesetzt. Bevorzugt sind Mengen von 0,1 bis 5,0, besonders bevorzugt 0,2 bis 1,0 Mol-%.

Die nach dem Verfahren herstellbaren ungesättigten Ester stellen wertvolle Comonomere für radikalisch initiierte Polymerisationen dar. Sie sind aufgrund der Einfachheit des Verfahrens erstmals auch im großtechnischen Maßstab zugänglich. Aufgrund der Labilität des 4-Chloratoms eignen sie sich zur Herstellung von 4-Amino- oder 4-Acyloxy-substituierten Acrylsäureestern.

## Beispiel 1

Man stellt aus 191,3 g Methacrylsäure (90%, 2 Mol) 331,5 g Wasser und 175,2 g 50%iger Natronlauge (2,2 Mol) unter Zusatz von 2,5 g N-Nitrosodiphenylamin, 2,5 g 4-Methoxyphenol und 8 g $(C_{12-18}H_{29-37})N^{\oplus}(CH_2CHOHCH_3)_2-CH_2C_6H_5)Cl^{\ominus}$ (70%ig) eine Lösung des Natriumsalzes der Methacrylsäure her und erwärmt diese auf 90°C. Dann werden innerhalb von 10 Minuten 750 g (6 Mol) 1,4-Dichlorbuten-2 unter Rühren zugegeben. Nach 1 Stunde Erwärmen bei der gleichen Temperatur

setzt man noch 8 g NaOH (50%ig) zu und läßt weitere 2 Stunden reagieren. Man kühlt ab und unterbricht das Rühren. Nach Trennung der Phasen wird die untere wäßrige Phase entfernt und die organische Phase im Vakuum unter Verwendung einer Füllkörperkolonne (Höhe 30 cm) destilliert. Als erste Fraktion werden bei 25 bis 58° C (2,3 mb) 530 g Dichlorbuten abgetrennt (4,24 Mol). Die zweite Fraktion (Sdp. 72 bis 80° C/2 mb) enthält das reine 4-Chlor-2-butenylmethacrylat. Es werden 242,2 g (=69,4% d. Th. △ 1,39 Mol) isoliert. Als Rückstand verbleiben 44,9 g einer dünnflüssigen Substanz, die aufgrund des Dünnschichtchromatogramms noch etwa 20 bis 30% des Esters enthält. Der destillierte Chlorbutenylester wird mit 50 ppm 4-Methoxyphenol stabilisiert. $n_D^{25}$ = 1.4808.

Analyse: $C_8H_{11}ClO_2$ (174,63)

| | | | | |
|---|---|---|---|---|
| ber.: | C 55,02 | H 6,35 | Cl 20,31 | O 18,32 |
| gef.: | C 55,0 | H 6,5 | Cl 20,3 | O 18,6 |

Beispiel 2

Analog Beispiel 1 wird der Chlorbutenylmethacrylsäureester jedoch unter Einsatz von nur 4 Mol (500 g) 1,4-Dichlorbuten-2 hergestellt. Nach Aufarbeitung gemäß Beispiel 1 erhält man 208,5 g Ester (59,6% d. Th.), 275 g Dichlorbuten (2,2 Mol) werden zurückgewonnen.

Beispiel 3

Analog Beispiel 1 wird die Umsetzung jedoch unter Einsatz von 8 Mol 1,4-Dichlorbuten-2 (1000 g) vorgenommen. Nach Aufarbeitung werden 271,4 g (77,7% d. Th.) Ester isoliert.

Beispiel 4

Aus 95,65 g (1 Mol) Methacrylsäure (90%), 87,6 g (50%) NaOH und 165,8 g Wasser wird eine Lösung des Na-Salzes der Methacrylsäure hergestellt. Man fügt 1,2 g N-Nitrosodiphenylamin und 1,2 g 4-Methoxyphenol zu und läßt unter Rühren bei 90° C 250 g (2 Mol) 1,4-Dichlorbuten-2 rasch zulaufen. Nach einer Stunde fügt man noch 4 g 50%ige Natronlauge zu und erhitzt dann unter Rühren weitere 2 Stunden. Man trennt die Phasen dann ab. In der wäßrigen Phase lassen sich 0,78 Mol NaCl argentometrisch nachweisen. Die organische Phase wird wie beschrieben destilliert. Erhalten werden neben 169,0 g (1,35 Mol) Dichlorbuten 67,5 g des 4-Chlorbutenylmethacrylats das sind 38,5% d. Th. Im dünnflüssigen Rückstand (6,8 g) befindet sich das Butendioldimethylacrylat.

Ohne Katalysator werden also deutlich schlechtere Ausbeuten erhalten.

Beispiel 5

Aus 216,2 g Acrylsäure (100%ig = 3 Mol), 262,8 g NaOH (50%ig) und 497 g Wasser wird eine wäßrige Lösung des Natriumacrylats bereitet, der man 3,7 g N-Nitrosodiphenylamin und 3,7 g 4-Methoxyphenol zur Stabilisierung zugibt. Ferner werden 12 g des in Beispiel 1verwendeten quartären Ammoniumsalzes zugefügt. Bei 90° C läßt man 1125 g (9 Mol) 1,4-Dichlorbuten zulaufen, gibt nach 1 Stunde 15,9 g $Na_2CO_3$ zu und läßt weitere 2 Stunden bei 90° C reagieren. Nach Phasentrennung wird die organische Schicht im Vakuum destilliert. Man erhält 286,0 g (1,78 Mol) 4-Chlor-2-butenylacrylat (59,4% d. Th.).

Zurückgewonnen werden 641 g (5,13 Mol) Dichlorbuten.

**Patentansprüche**

1. Verfahren zur Herstellung von Halogenbutenylacrylaten der Formel (I)

$$\underset{\displaystyle R}{\overset{}{H_2C=\underset{|}{C}}}-COOCH_2-CH=CH-CH_2-X \qquad (I)$$

in der

R Wasserstoff oder einen Alkylrest mit 1 bis 4 Kohlenstoffatomen und
X ein Halogenatom darstellen,

dadurch gekennzeichnet, daß man pro Mol eines Salzes der Acrylsäure gemäß Formel (II)

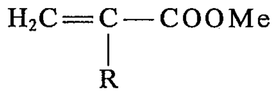

(II)

in der

R Wasserstoff oder einen Alkylrest mit 1 bis 4 Kohlenstoffatomen und
Me ein Ammonium, ein Alkali- oder Erdalkalimetallatom-Äquivalent darstellen,

1 bis 7 Mol an 1,4-Dihalogenbuten-(2) in einem Zweiphasensystem bei Temperaturen von 10 bis 140°C miteinander umsetzt.

2. Verfahren gemäß Anspruch 1, dadurch gekennzeichnet, daß die Reaktionstemperatur 60 bis 100°C beträgt.

3. Verfahren gemäß Ansprüchen 1 und 2, dadurch gekennzeichnet, daß die Reaktionstemperatur 80 bis 95°C beträgt.

4. Verfahren gemäß Ansprüchen 1 bis 3, dadurch gekennzeichnet, daß pro Mol eines Salzes der Acrylsäure gemäß Formel (II) 2 bis 5 Mol an 1,4-Dihalogenbuten-(2) eingesetzt werden.

5. Verfahren gemäß Ansprüchen 1 bis 4, dadurch gekennzeichnet, daß die Reaktion in Gegenwart eines Phasentransfer-Katalysators aus der Gruppe der quartären Ammonium-, Phosphonium- oder Sulfoniumsalze durchgeführt wird.

6. Verfahren gemäß Anspruch 5, dadurch gekennzeichnet, daß der Phasentransfer-Katalysator in Mengen von 0,01 bis 10 Mol-% eingesetzt wird.

7. Verfahren gemäß Ansprüchen 5 und 6, dadurch gekennzeichnet, daß der Phasentransferkatalysator in Mengen von 0,1 bis 5 Mol-% anwesend ist.

8. Verfahren gemäß Anspruch 5, dadurch gekennzeichnet, daß der Phasentransfer-Katalysator in Mengen von 0,2 bis 1 Mol-% anwesend ist.

**Claims**

1. Process for the preparation of halogen butenyl acrylates of the formula (I)

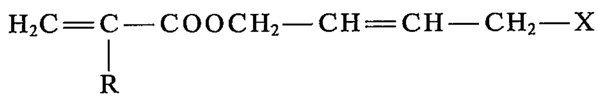

(I)

wherein

R represents hydrogen or a $C_1-C_4$ alkyl group; and
X represents a halogen atom;

characterised in that a salt of acrylic acid of the formula (II)

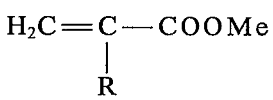

(II)

wherein

R represents hydrogen or a $C_1-C_4$ alkyl group; and
Me represents one equivalent of an ammonium, an alkali metal or alkaline earth metal atom;

is reacted with 1,4-dihalogen butene-(2) in proportions of 1 mol of acrylic acid salt to from 1 to 7 mol of the dihalogen butene in a diphasic system at temperatures of from 10 to 140°C.

2. Process according to claim 1, characterised in that the reaction temperature is from 60 to 100°C.

3. Process according to claims 1 and 2, characterised in that the reaction temperature is from 80 to 95°C.

4. Process according to claims 1 to 3, characterised in that from 2 to 5 mol of 1,4-dihalogen butene-(2) are used per mol of a salt of acrylic acid corresponding to formula (II).

5. Process according to claims 1 to 4, characterised in that the reaction is carried out in the presence of a phase transfer catalyst selected from quaternary ammonium, phosphonium and sulphonium salts.

6. Process according to claim 5, characterised in that the phase transfer catalyst is used in quantities of from 0.01 to 10 mol percent.

7. Process according to claims 5 and 6, characterised in that the phase transfer catalyst is present in quantities of from 0.1 to 5 mol percent.

8. Process according to claim 5, characterised in that the phase transfer catalyst is present in quantities of from 0.2 to 1 mol percent.

**Revendications**

1. Procédé de préparation d'acrylates d'halogénobutényle de formule (I):

$$H_2C=\underset{\displaystyle R}{\underset{|}{C}}-COOCH_2-CH=CH-CH_2-X \qquad (I)$$

dans laquelle:

R représente l'hydrogène ou un groupe alkyle en $C_1-C_4$, et
X représente un atome d'halogène,

caractérisé en ce que l'on fait réagir entre elles, à une température de 10 à 140° C dans un système à deux phases, 1 mole d'un sel de l'acide acrylique de formule (II):

$$H_2C=\underset{\displaystyle R}{\underset{|}{C}}-COOMe \qquad (II)$$

dans laquelle:

R représente l'hydrogène ou un groupe alkyle en $C_1-C_4$, et
Me représente un groupe ammonium, un équivalent de métal alcalin ou alcalino-terreux,

et 1 à 7 moles de 1,4-dihalogénobutène-(2).

2. Procédé selon la revendication 1, caractérisé en ce que la température de réaction est de 60 à 100° C.

3. Procédé selon les revendications 1 et 2, caractérisé en ce que la température de réaction est de 80 à 95° C.

4. Procédé selon les revendications 1 à 3, caractérisé en ce que, pour 1 mole d'un sel de l'acide acrylique de formule (II), on utilise 2 à 5 moles de 1,4-dihalogénobutène-(2).

5. Procédé selon les revendications 1 à 4, caractérisé en ce que la réaction est effectuée en présence d'un catalyseur de transfert de phase pris dans le groupe des sels d'ammonium ou de phosphonium quaternaires ou de sulfonium.

6. Procédé selon la revendication 5, caractérisé en ce que l'on utilise le catalyseur à transfert de phase en quantité de 0,01 à 10 moles %.

7. Procédé selon les revendications 5 et 6, caractérisé en ce que le catalyseur à transfert de phase est présent en quantité de 0,1 à 5 moles %.

8. Procédé selon la revendication 5, caractérisé en ce que le catalyseur à transfert de phase est présent en quantité de 0,2 à 1 mole %.